(19) Europäisches Patentamt
European Patent Office
Office européen des brevets

(11) Numéro de publication : **0 078 735**
**B1**

(12) # FASCICULE DE BREVET EUROPÉEN

(45) Date de publication du fascicule du brevet : **18.09.85**

(21) Numéro de dépôt : **82401959.0**

(22) Date de dépôt : **25.10.82**

(51) Int. Cl.[4] : **C 07 D307/00**, A 61 K 31/34// C07D407/12

(54) **Nouveaux dérivés appartenant à la famille des 3-amino-1-hétéroaryloxy-2-propanols, utilisation en thérapeutique et procédé de préparation.**

(30) Priorité : **30.10.81 FR 8120471**

(43) Date de publication de la demande : **11.05.83 Bulletin 83/19**

(45) Mention de la délivrance du brevet : **18.09.85 Bulletin 85/38**

(84) Etats contractants désignés : **AT BE CH DE FR GB IT LI LU NL SE**

(56) Documents cités : **FR-A- 2 137 901**

(73) Titulaire : **LABORATOIRES DEBAT Société anonyme dite:**
**60 rue de Monceau**
**F-75008 Paris (FR)**

(72) Inventeur : **Lemoine, Jean**
**7 Suite de la Portaille**
**F-92380 Garches (FR)**
Inventeur : **Riffaud, Jean-Pierre**
**48 rue Albert Joly**
**F-78000 Versailles (FR)**

(74) Mandataire : **Clisci, Serge et al**
**CABINET BEAU DE LOMENIE 55 rue d'Amsterdam**
**F-75008 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

EP 0 078 735 B1

**Description**

La présente invention concerne, en tant que produits industriels, de nouveaux dérivés appartenant à la famille des 3-amino-1-hétéroaryloxy-2-propanols, à savoir des dérivés du type 2,4-diacétyl-5-(3-alkylamino-2-hydroxy-propyloxy)-benzofuranne. Elle concerne également l'utilisation en thérapeutique et le procédé de préparation de ces nouveaux dérivés.

On sait que l'on a déjà préconisé, en tant qu'agents β-bloquants, des substances appartenant à l'ensemble des 3-amino-1-aryloxy-2-propanols et 3-amino-1-hétéroaryloxy-2-propanols. On sait, en particulier, que le propanolol (ou 3-isopropylamino-1-α-naphtyloxy-2-propanol), qui est notamment connu des brevets français n° 1 503 510 et n° 4 057 M, est un excellent β-bloquant de référence agissant simultanément sur les récepteurs $\beta_1$ (récepteurs cardiaques) et les récepteurs $\beta_2$ (récepteurs bronchiques). On sait également que les dérivés de benzofuranne selon le brevet français n° 72-17 290 (publication n° 2 137 901), qui sont monoacétylés et non diacétylés comme ceux de l'invention, ont des propriétés β-bloquantes qui ne sont pas cardiosélectives. On vient de trouver de façon surprenante que les nouveaux dérivés selon l'invention, qui sont structurellement différents des produits antérieurement connus, présentent l'avantage d'être des agents β-bloquants cardiosélectifs en ce sens qu'ils agissent sur les récepteurs $\beta_1$ et sont dénués d'effet sur les récepteurs $\beta_2$.

Les nouveaux dérivés selon l'invention qui appartiennent à la famille des 3-amino-1-hétéroaryloxy-2-propanols sont caractérisés en ce qu'ils sont choisis parmi l'ensemble constitué par :

(i) les (±)-, (+)- et (−)-2,4-diacétyl-5-(3-alkyl-amino-2-hydroxy-propyloxy)-benzofurannes répondant à la formule générale :

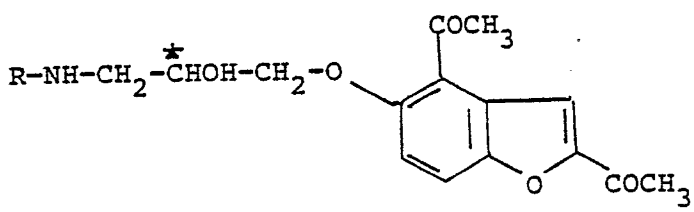

(I)

(où R représente un groupe isopropyle ou tertiobutyle), et

(ii) leurs sels d'addition d'acide.

L'invention englobe donc les racémiques (±) de formule I ainsi que les isomères optiques (+) et (−).

Parmi les acides minéraux et organiques qui conviennent pour salifier les bases libres de formule I, on peut notamment citer les acides chlorhydrique, maléique, fumarique, aspartique et paratoluènesulfonique.

Les composés de formule I peuvent être préparés selon une méthode connue en soi par application de mécanismes réactionnels classiques. Le procédé que l'on préconise selon l'invention est schématisé par le diagramme I ci-après.

Ce procédé comprend successivement :

a) la réaction du 2,4-diacétyl-5-hydroxybenzofuranne II [préparé comme indiqué par Jean-Marc Clavel et al., Bull. Soc. Chim., 1976, pages 131-134] avec un époxyde halogéné de formule III (où Hal est Cl ou Br, l'halogène préféré étant le brome), pour obtenir le 2,4-diacétyl-5-(2,3-époxy-propyloxy)-benzofuranne IV ; et

b) la réaction du composé IV ainsi obtenu avec une amine $NH_2R$ (où R est isopropyle ou tertiobutyle) pour obtenir un composé de formule I

DIAGRAMME I

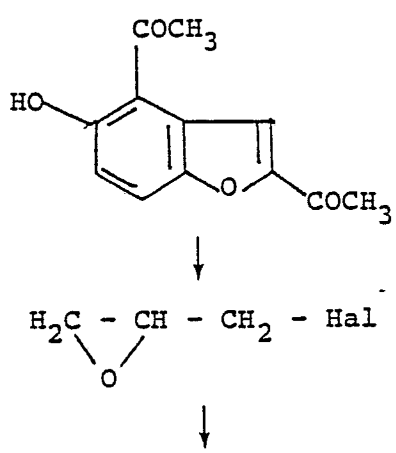

(II)

(III)

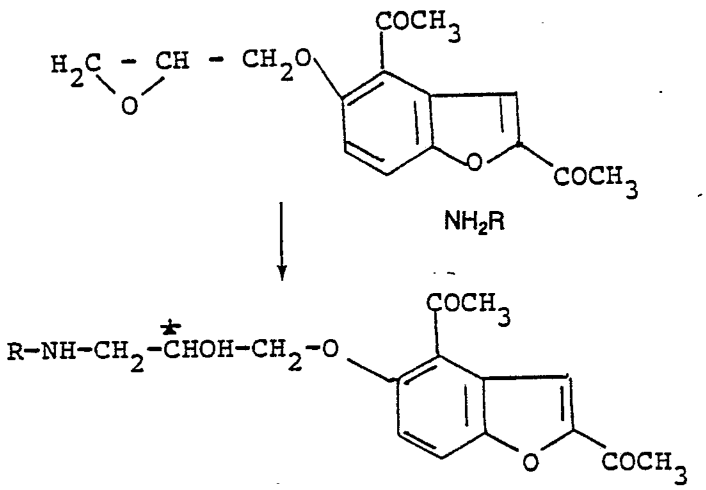


(IV)

(I)

Le meilleur mode de mise en œuvre de ce prodédé consiste à faire réagir :

— au stade a) 1,2 à 1,8 moles de III, avec 1 mole de II dans une cétone en $C_3$-$C_6$ (notamment l'acétone, la 2-butanone, la 2-pentanone, la 3-pentanone), en présence d'un composé choisi parmi Li, Na, K, $Na_2CO_3$ et $K_2CO_3$, au reflux pendant au moins 4 heures ; et

— au stade b) 1,5 à 2,2 moles de $NH_2R$ avec 1 mole de IV, dans un alcool inférieur en $C_1$-$C_3$, au reflux pendant au moins 2 heures.

Les énantiomères de formule I peuvent être résolus à partir du racémique selon une méthode connue en soi.

Les produits selon l'invention sont des agents β-bloquants cardiosélectifs en ce sens qu'ils agissent sur les récepteurs $\beta_1$ sans avoir d'effet sur les récepteurs $\beta_2$. En raison de leurs propriétés anti-arythmisantes, anti-hypertensives et anti-angor, ils sont indiqués dans le traitement des maladies cardiovasculaires telles que notamment l'hypertension artérielle, la diminution ou la prévention des troubles du rythme, la prophylaxie de l'angine de poitrine et les manifestations cardiovasculaires des hyperthyroïdies.

Selon l'invention, on préconise une composition thérapeutique qui est caractérisée en ce qu'elle renferme, en association avec un excipient physiologiquement acceptable, au moins un composé de formule I ou l'un de ses sels d'addition d'acide.

D'autres avantages et caractéristiques de l'invention seront mieux compris à la lecture qui va suivre d'exemples de préparation nullement limitatifs mais donnés à titre d'illustration.

## Préparation I

Obtention du chlorhydrate de (±)-2,4-diacétyl-5-(3-tertiobutylamino-2-hydroxy-propyloxy)-benzofuranne

(Exemple 1 ; n° de code R 7262)

a) 2,4-Diacétyl-5-(2,3-époxy-propoloxy)-benzofuranne

Dans un ballon, on maintient au reflux pendant 8 heures et sous agitation une suspension de 10 g (0,046 mole) de 2,4-diacétyl-5-hydroxybenzofuranne, de 9,5 g (0,069 mole) de $K_2CO_3$ sec et de 9,45 g (0,069 mole) d'épibromhydrine dans 100 ml de 2-butanone préalablement séchée. On élimine par filtration les sels minéraux (KBr formé et $K_2CO_3$ restant) après les avoir rincés à l'acétone. Le filtrat résultant est évaporé à sec, et le résidu d'évaporation est repris avec le chloroforme. La phase chloroformique ainsi obtenue est lavée à l'eau et séchée sur $MgSO_4$. On élimine le chloroforme par évaporation à sec. Par recristallisation du résidu d'évaporation dans le mélange benzène-cyclohexane (1 : 1) v/v, on obtient 11,8 g (rendement : 93 %) de produit attendu. F = 114 °C.

b) (±)-2,4-Diacétyl-5-(3-tertiobutylamino-2-hydroxy-propyloxy)-benzofuranne

Dans un ballon, on mélange 10,5 g (0,0383 mole) de 2,4-diacétyl-5-(2,3-époxy-propyloxy)-benzofuranne, 5,6 g (0,0767 mole) de tertiobutylamine et 100 ml d'éthanol. On porte au reflux pendant 4 heures. On évapore ensuite l'éthanol. Par recristallisation dans le mélange toluène-cyclohexane (1 : 1) v/v à − 30 °C, on obtient 9 g (rendement : 70 %) de produit attendu. F = 122 °C.

c) Chlorhydrate de (±)-2,4-diacétyl-5-(3-tertiobutylamino-2-hydroxypropyloxy)-benzofuranne

Dans un réacteur refroidi extérieurement avec un bain de glace, on charge 8,9 g de (±)-2,4-diacétyl-5-(3-tertiobutyl-amino-2-hydroxy-propyloxy)-benzofuranne en solution dans $CHCl_3$, puis fait barboter un courant de HCl gazeux jusqu'à saturation. Après évaporation de $CHCl_3$ sous pression réduite, on obtient un précipité que l'on reprend dans $CHCl_3$, triture, essore et sèche. On obtient 9,2 g de produit attendu. F = 214-216 °C.

Preparation II

Obtention du chlorhydrate de (±)-2,4-diacétyl-5-(3-isopropylamino-2-hydroxy-propyloxy)-benzofuranne

(Exemple 2)

En procédant comme indiqué dans la préparation I, mais en remplaçant au stade b) la tertiobutylamine par l'isopropylamine, on obtient le chlorhydrate de (±)-2,4-diacétyl-5-(3-isopropylamino-2-hydroxy-propyloxy)-benzofuranne.

On a résumé ci-après une partie des résultats des essais qui ont été entrepris chez l'animal, en particulier avec le produit préféré selon l'invention, à savoir le R 7262 (exemple 1).

Toxicité

Par voie orale, la DL-50 du produit de l'exemple 1 est supérieure à 500 mg/kg chez la souris.

En ce qui concerne la DL-O (dose maximale non mortelle) chez le cobaye par voie intraveineuse, on constate au tableau I ci-après que le produit de l'exemple 1 est moins toxique que le propanolol.

Tableau I

| Produit | N° de code | DL-O iv cobaye mg/kg |
|---|---|---|
| Exemple 1 | R 7262 | 48,3 |
| Propanolol | | 26,1 |

Etude des propriétés β-bloquantes in vivo chez le cobaye

a) Etude au niveau cardiaque

Des cobayes mâles, tricolores, d'un poids moyen de 400 à 500 g, sont anesthésiés à l'éthyl-uréthane (1,5 g/kg, voie i.p.). Le rythme cardiaque est enregistré à partir du signal pulsatile de la pression artérielle recueillie à la carotide gauche.

Après une période de stabilisation de 30 minutes, l'effet tachycardisant est déterminé par une injection i.v. d'isoprénaline à 0,25 ug/kg avant puis 15, 30, 45 et 60 minutes après l'injection du β-bloquant à étudier ou de son solvant. Cet effet est exprimé par le rythme cardiaque atteint par rapport au rythme initial. Les résultats ont été consignés dans le tableau II ci-après.

b) Etude au niveau bronchique

L'étude sur les récepteurs bronchiques $\beta_2$ a été réalisée sur les animaux utilisés pour l'étude au niveau cardiaque, dans le même temps que celle-ci.

L'effet $\beta_2$-bloquant des substances à étudier a été recherché selon la méthode décrite par LINEE et al., (1974) à partir de la technique de KONZETT et ROESSLER (1940). Dans ce but, on a mesuré la variation, provoquée par l'administration d'isoprénaline et des β-bloquants à étudier, de l'amplitude du bronchospasme induit par la sérotonine. Ici, l'isoprénaline a été introduite 30 secondes avant l'injection i.v. de sérotonine puis l'amplitude du bronchospasme a été mesurée avant puis 15, 30, 45 et 60 minutes après l'injection du β-bloquant à étudier ou de son solvant. Les résultats ont été consignés dans le tableau III ci-après.

Les résultats des tableaux II et III montrent que le R 7262 est un agent β-bloquant cardiosélectif.

(Voir Tableaux pages suivantes)

Tableau II

Effet bloquant sur les récepteurs cardiaques $\beta_1$

| Produit | nombre d'animaux traités | dose mg/kg | Rythme cardiaque initial | Rythme cardiaque provoqué par injection d'isoprénaline | | | | |
|---|---|---|---|---|---|---|---|---|
| | | | | avant administration de β-bloquant | Après administration (temps en minutes) de β-bloquant | | | |
| | | | | | 15 min | 30 min | 45 min | 60 min |
| (témoins) | 10 | - | 227 ± 17 | 268 ± 21 | 257 ± 17 | 251 ± 18 | 245 ± 18 | 240 ± 17 |
| Propanolol | 6 | 0,0625 | 262 ± 44 | 289 ± 39 | 255 ± 49 | 269 ± 48 | 272 ± 48 | 274 ± 51 |
| Propanolol | 6 | 0,25 | 202 ± 28 | 257 ± 36 | 209 ± 35 | 228 ± 45 | 231 ± 49 | 232 ± 48 |
| Exemple 1 (R 7262) | 7 | 0,0625 | 245 ± 33 | 293 ± 28 | 266 ± 22 | 275 ± 24 | 283 ± 22 | 285 ± 20 |
| Exemple 1 (R 7262) | 7 | 0,25 | 223 ± 45 | 257 ± 36 | 217 ± 48 | 228 ± 51 | 237 ± 51 | 241 ± 51 |

Note les résultats sont exprimés sous la forme :
Moyenne ± Ecart type à la moyenne

Tableau III

Effet bloquant sur les récepteurs bronchiques $\beta_2$

| Produit | Nombre d'animaux traités | Dose mg/kg | AMPLITUDE DU BRONCHOSPASME * | | | | |
|---|---|---|---|---|---|---|---|
| | | | Avant produit β-bloquant | Après produit β-bloquant | | | |
| | | | | 15 min | 30 min | 45 min | 60 min |
| - (témoins) | 10 | - | 74 ± 11 | 62 ± 13 | 57 ± 12 | 69 ± 11 | 68 ± 13 |
| Propanolol | 6 | 0,0625 | 79 ± 9 | 8 ± 5 | 12 ± 8 | 16 ± 8 | 22 ± 8 |
| Propanolol | 6 | 0,25 | 88 ± 5 | 2 ± 1 | 3 ± 1 | 3 ± 1 | 7 ± 3 |
| Ex 1 (R 7262) | 7 | 0,0625 | 76 ± 9 | 58 ± 13 | 54 ± 10 | 43 + 11 | 54 ± 13 |
| Ex 1 (R 7262) | 7 | 0,25 | 72 ± 11 | 58 ± 10 | 45 ± 11 | 45 ± 13 | 51 ± 11 |

Note

* l'amplitude du bronchospasme est exprimée en % par rapport à l'amplitude du bronchospasme induit par la sérotonine ; les résultats sont donnés ici sous la forme : Moyenne ± Ecart type à la moyenne

Etude des propriétés β-bloquantes chez le chien anesthésié

Des chiens bâtards, de l'un ou l'autre sexe, pesant de 13 à 30 kg ont été utilisés. L'anesthésie est induite par injection i.p. de Nembutal (25 mg/kg) et maintenue par une perfusion de Nembutal (25 mg/kg au rythme de 0,2 ml/min). La trachée est intubée et l'animal est placé sous assistance respiratoire (respirateur du type « BIRD MARK 8 »). La pression artérielle carotidienne est mesurée par un capteur de pression (du type « NARCO P 1000 B »). Après thoracotomie, la pression ventriculaire gauche est mesurée au moyen d'un capteur de même type. Le rythme cardiaque est enregistré, à partir du signal de la pression carotidienne, par un cardiotachymètre (« NARCO type 7302 »). La vitesse de croissance de la tension isométrique du ventricule gauche (dP/dt) a été évaluée par un coupleur différentiateur (« NARCO type 7301 »).

Après injection par voie i.v. d'atropine (2 mg/kg) et une période de stabilisation de 30 minutes, les courbes action-dose de l'isoprénaline, pour chacun des paramètres, ont été recherchées. Les injections de chaque dose d'isoprénaline ont été espacées de 5 minutes. Après une période de 10 minutes, les animaux reçoivent la première dose de β-bloquant puis 10 minutes après la série des doses d'isoprénaline. De nouveau 10 minutes après, l'injection de la seconde dose d'isoprénaline est pratiquée. Cette séquence expérimentale est répétée jusqu'à ce que chaque animal ait reçu 3 à 4 doses de β-bloquant.

L'évaluation de l'antagonisme des effets de l'isoprénaline a été déterminée par le calcul du $pA_{10}$ qui est la dose d'antagoniste, en mg/kg, en présence de laquelle il faut multiplier les doses d'isoprénaline par 10 (« dose ratio 10 ») pour avoir le même effet qu'en l'absence d'antagoniste. Le $pA_{10}$ a été calculé par extrapolation de la courbe $\log (R \times D^{-1}) = f(pA_X)$ où $R \times D^{-1}$ est le rapport des doses équiactives de l'isoprénaline.

Les effets de l'isoprénaline ont été mesurés par rapport aux valeurs de base prises juste avant le début de chaque nouvelle gamme d'isoprénaline en ce qui concerne la fréquence cardiaque et la dP/dt. Pour la pression artérielle diastolique, en raison de l'activité hypotensive marquée du R 7262, les effets de l'isoprénaline ont été évalués par rapport à la valeur initiale, prise avant toute injection.

Les résultats sont consignés dans le tableau IV.

Tableau IV

Effet β-bloquant chez le chien anesthésié

| Produit (nombre d'animaux) | Fréquence cardiaque | | P.A. diastolique | | dP/dt | |
|---|---|---|---|---|---|---|
| | $pA_{10}$ | pente | $pA_{10}$ | pente | $pA_{10}$ | pente |
| R 7262 (n = 5) | 0,23 | 0,7. | 3 | - | 0,10 | 0,9 |
| Propanolol (n = 5) | 0,24 | 1,1 | 0,1 | - | 0,13 | 0,8 |

On constate des résultats du tableau IV que, chez le chien anesthésié, le R 7262 antagonise les effets de l'isoprénaline au niveau des récepteurs $\beta_1$ : action chronotrope et inotrope positives. En revanche, le R 7262 n'a aucune activité vis-à-vis des effets $\beta_2$ de l'isoprénaline qui sont illustrés ici par l'action hypotensive. Il y a donc cardiosélectivité pour le R 7262 à la différence du propanolol.

**Revendications** (pour les Etats contractants : BE, CH, DE, FR, GB, IT, LI, LU, NL, SE)

1. Nouveau dérivé appartenant à la famille des 3-amino-1-hétéroaryloxy-2-propanols, caractérisé en ce qu'il est choisi parmi l'ensemble constitué par :

(i) les (±)-, (+)- et (−)-2,4-diacétyl-5-(3-alkylamino-2-hydroxy-propyloxy)-benzofurannes répondant à la formule générale :

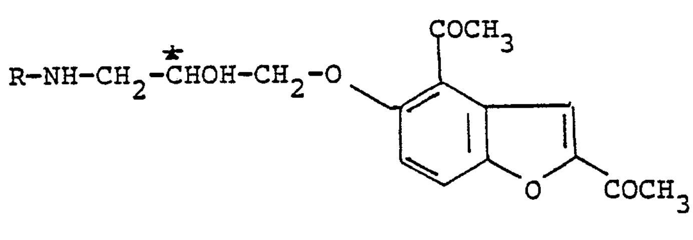

(I)

(où R représente un groupe isopropyle ou tertiobutyle), et

(ii) leurs sels d'addition d'acide.

2. (±)-2,4-Diacétyl-5-(3-tertiobutylamino-2-hydroxy-propyloxy)-benzofuranne et ses sels d'addition d'acide.

3. (±)-2,4-Diacétyl-5-(3-isopropylamino-2-hydroxy-propyloxy)-benzofuranne et ses sels d'addition d'acide.

4. Composition thérapeutique, caractérisée en ce qu'elle renferme, en association avec un excipient physiologiquement acceptable, au moins un composé selon l'une quelconque des revendications 1 à 3.

5. Procédé de préparation d'un composé de formule I selon la revendication 1 à partir du 2,4-diacétyl-5-hydroxybenzofuranne, caractérisé en ce que

a) on fait réagir le 2,4-diacétyl-5-hydroxybenzofuranne de formule :

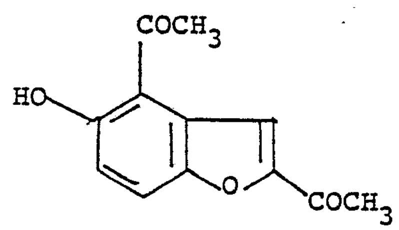

(II)

avec un époxyde halogéné de formule :

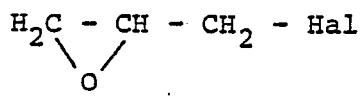

(III)

(où Hal représente Cl ou Br) pour obtenir le 2,4-diacétyl-5-(2,3-époxy-propyloxy)-benzofuranne de formule :

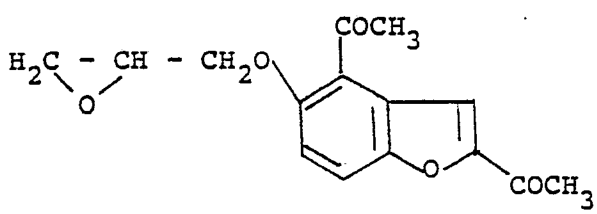

(IV)

puis,

b) on fait réagir le dérivé IV ainsi obtenu avec une amine $NH_2R$ (où R est défini comme dans la revendication 1).

6. Procédé selon la revendication 5, caractérisé en ce que :

a) on fait réagir 1,2 + 1,8 moles de III avec 1 mole de II dans une cétone en $C_3$-$C_6$ en présence d'un composé choisi parmi Li, Na, K, $Na_2CO_3$ et $K_2CO_3$, au reflux pendant au moins 4 heures ; et

b) on fait réagir 1,5 à 2,2 moles de $NH_2R$ (où R est défini comme dans la revendication 1) avec 1 mole de IV dans un alcool en $C_1$-$C_3$, au reflux pendant au moins 2 heures.

**Revendications** (pour l'Etat contractant AT)

1. Procédé pour la préparation d'un nouveau dérivé appartenant à la famille des 3-amino-1-hétéroaryloxy-2-propanols et choisi parmi l'ensemble constitué par les (±)-, (+)- et (−)-2,4-diacétyl-5-(3-alkylamino-2-hydroxy-propyloxy)-benzofurannes répondant à la formule générale :

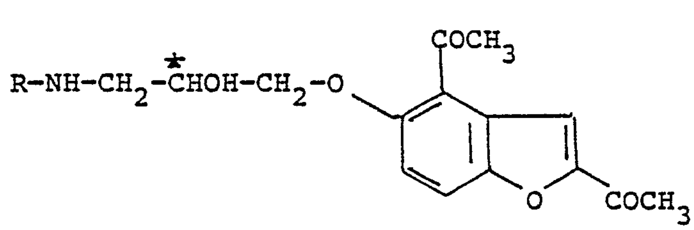

(I)

(où R représente un groupe isopropyle ou tertiobutyle), et leurs sels d'addition d'acide, ledit procédé étant caractérisé en ce que :

a) on fait réagir le 2,4-diacétyl-5-hydroxybenzofuranne de formule :

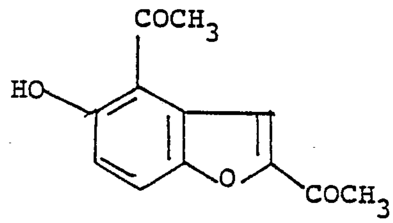

(II)

avec un époxyde halogéné de formule :

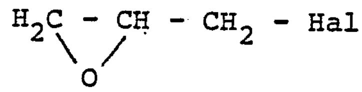

(III)

(où Hal représente Cl ou Br) pour obtenir le 2,4-diacétyl-5-(2,3-époxy-propyloxy)-benzofuranne de formule :

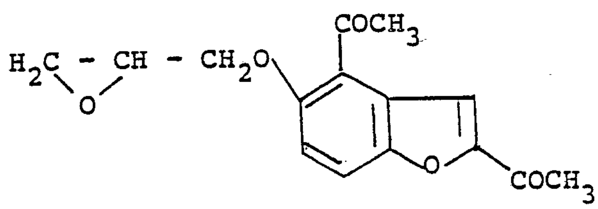

(IV)

puis,

b) on fait réagir le dérivé IV ainsi obtenu avec une amine $NH_2R$ (où R est défini comme ci-dessus).

2. Procédé selon la revendication 1, caractérisé en ce que :

a) on fait réagir 1,2 à 1,8 moles de III avec 1 mole de II dans une cétone en $C_3$-$C_6$ en présence d'un composé choisi parmi Li, Na, K, $Na_2CO_3$ et $K_2CO_3$, au reflux pendant au moins 4 heures ; et

b) on fait réagir 1,5 à 2,2 moles de $NH_2R$ (où R est défini comme ci-dessus) avec 1 mole de IV dans un alcool en $C_1$-$C_3$, au reflux pendant au moins 2 heures.

3. Utilisation des produits tels qu'obtenus selon l'une des revendications précédentes et ayant pour formule (I) pour la préparation des compositions thérapeutiques actives comme médicaments β bloquants.

**Claims** (for the Contracting States : BE, CH; DE, FR, GB, IT, LI, LU, NL, SE)

1. A new derivative belonging to the family of 3-amino-1-hetero-aryloxy-2-propanols, characterized in that it is selected from the group consisting of :

(i) the (±)-, (+)-2,4-diacetyl-5-(3-alkyl-amino-2-hydroxypropyloxy)-benzofurans of the general formula :

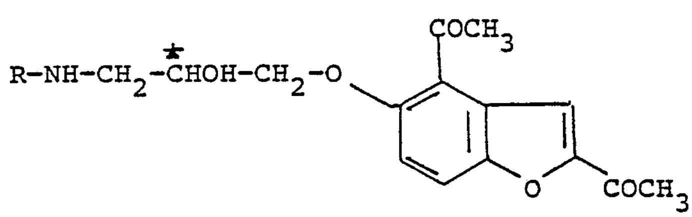

(I)

(wherein R represents an isopropyl or tertiobutyl group), and

(ii) the acid addition salts thereof.

2. (±)-2,4-Diacetyl-5-(3-tertiobutylamino-2-hydroxy-propyloxy)-benzofuran and its acid addition salts.

3. (±)-2,4-Diacetyl-5-(3-isopropylamino-2-hydroxy-propyloxy)-benzofuran and its acid addition salts.

4. A therapeutical composition, characterized in that it contains, in association with a physiologically acceptable excipient, at least one compound according to any one of claims 1 to 3.

5. A method for preparing a compound of formula I according to claim 1, from 2,4-diacetyl-5-hydroxy-benzofuran, characterized in that it comprises the steps of :

a) reacting the 2,4-diacetyl-5-hydroxy-benzofuran of formula :

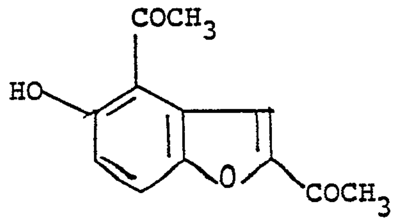

(II)

with a halogenated epoxide of formula :

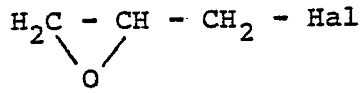

(III)

(wherein Hal represents Cl ou Br) to obtain the 2,4-diacetyl-5,2,3-epoxypropyloxy)-benzofuran of the formula :

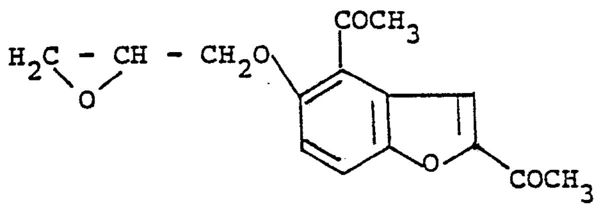

(IV)

then

b) reacting the derivative IV thus obtained with an amine $NH_2R$ (wherein R is defined as hereinabove).

6. The method according to claim 5, characterized in that :

a) 1.2 to 1.8 moles of III are reacted with 1 mole of II in a ketone having from 3 to 6 C atoms in the presence of a compound selected from Li, Na, K, $Na_2CO_3$ and $K_2CO_3$, at reflux for at least 4 hours, and

b) 1.5 to 2.2 moles of $NH_2R$ (where R is defined as in claim 1) are reacted with 1 mole of IV in an alcohol having from 1 to 3 C atoms, at reflux for at least 2 hours.

**Claims** (for the Contracting State AT)

1. A method for the preparation of new (±)-, (+)- and (−)-2,4-diacetyl-5-(3-alkyl-amino-2-hydroxypropyloxy)-benzofurans of the general formula :

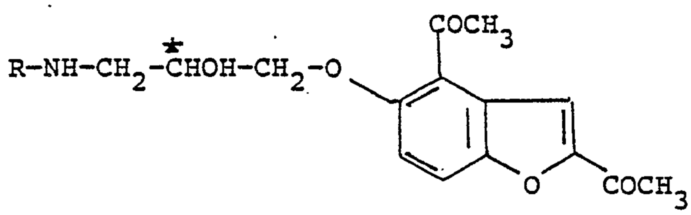

(I)

wherein R represents an isopropyl or tertiobutyl group as well as the acid addition salts thereof from 2,4-di-acetyl-5-hydroxybenzofuran, characterized in that

a) the 2,4-diacetyl-5-hydroxybenzofuran of formula :

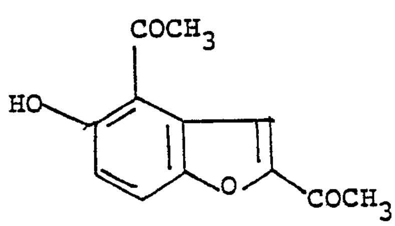

(II)

is reacted with a halogenated epoxide of formula :

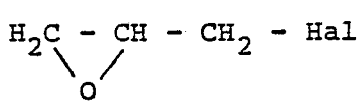

(III)

(wherein Hal represents Cl or Br) to obtain the 2,4-di-acetyl-5-(2,3)epoxypropyloxy)-benzofuran of the formula :

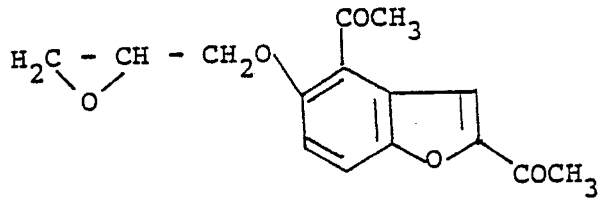

(IV)

and then,

b) the derivative IV thus obtained is reacted with an amine $NH_2R$ (wherein R is defined as hereinabove).

2. The method according to claim 1, characterized in that :

a) 1.2 to 1.8 moles of an epoxide of formula III are left to be reacted with 1 mole of the substituted benzofuran of formula II in a ketone having from 3 to 6 C atoms in the presence of a reagent selected from Li, Na, K, $Na_2CO_3$ and $K_2CO_3$, at reflux for at least 4 hours ; and

b) 1.5 to 2.2 moles of an amine of formula $NH_2R$ wherein R is defined as in claim 1 are left to be reacted with 1 mole of the compound of formula IV in an alcohol having from 1 to 3 C atoms, at reflux for at least 2 hours.

3. Application of the new compounds obtainable according to claims 1 and 2 of general formula (I) for the preparation of therapeutic compositions, acting as cardioselective β-blocking agents, said latter containing at least one new compound of formula (I) together with a physiologically acceptable excipient.

**Patentansprüche** (für die Vertragsstaaten : BE, CH, DE, FR, GB, IT, LI, LU, NL, SE)

1. Neues zur Familie der 3-Amino-1-heteroaryloxy-2-propanole gehörendes Derivat, dadurch gekennzeichnet, daß es ausgewählt ist aus der Gruppe, bestehend aus :

(i) den (±)-, (+)- und (−)-2,4-Diacetyl-5-(3-alkyl-amino-2-hydroxy-propyloxy)-benzofuranen der allgemeinen Formel

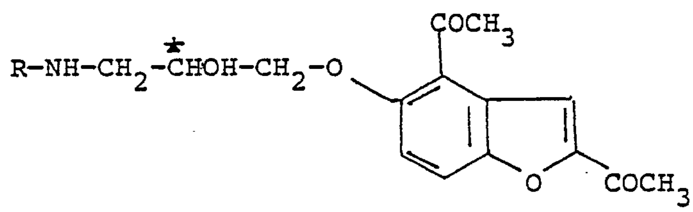

(I)

(worin R eine Isopropyl- oder tert.Butylgruppe darstellt) und

(ii) ihren Säureadditionssalzen.

2. (±)-2,4-Diacetyl-5-(3-tert.butylamino-2-hydroxy-propyloxy)-benzofuran und seine Säureadditionssalze.

3. (±)-2,4-Diacetyl-5-(3-isopropylamino-2-hydroxy-propyloxy)-benzofuran und seine Säureadditionssalze.

4. Therapeutische Zusammensetzung, dadurch gekennzeichnet, daß sie in Verbindung mit einem physiologisch akzeptablen Exzipienten zumindest eine Verbindung nach einem der Ansprüche 1 bis 3 enthält.

5. Verfahren zur Herstellung einer Verbindung der Formel I nach Anspruch 1 aus 2,4-Diacetyl-5-hydroxybenzofuran, dadurch gekennzeichnet, daß

a) 2,4-Diacetyl-5-hydroxybenzofuran der Formel

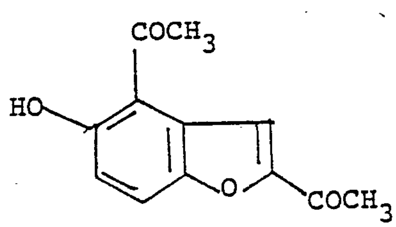

(II)

mit einem halogenierten Epoxid der Formel :

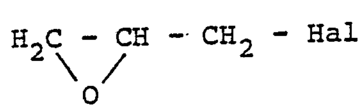

(III)

(worin Hal für Cl oder Br steht) zur Umsetzung gebracht wird, um das 2,4-Diacetyl-5-(2,3-epoxypropyloxy)-benzofuran der Formel

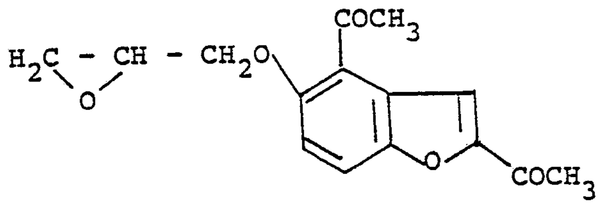

(IV)

zu erhalten, und danach

b) das so erhaltene Derivat IV mit einem Amin $NH_2R$ (worin R die Bedeutung des Anspruchs 1 hat) zur Umsetzung gebracht wird.

6. Verfahren nach Anspruch 5, dadurch gekennzeichnet, daß

a) 1,2 bis 1,8 Mol von III mit 1 Mol von II in einem $C_3$-$C_6$-Keton in Gegenwart einer Verbindung, ausgewählt aus Li, Na, K, $Na_2CO_3$ und $K_2CO_3$, wenigstens 4 h unter Rückfluß reagieren gelassen wird ; und

b) 1,5 bis 2,2 Mol von $NH_2R$ (worin R die Bedeutung des Anspruchs 1 hat) mit 1 Mol von IV in einem $C_1$-$C_3$-Alkohol wenigstens 2 h unter Rückfluß reagieren gelassen wird.

**Patentansprüche** (für den Vertragsstaat AT)

1. Verfahren zur Herstellung von neuen (±)-, (+)- und (−)-2,4-Diacetyl-5-(3-alkylamino-2-hydroxypropyloxy)-benzofuranen der allgemeinen Formel

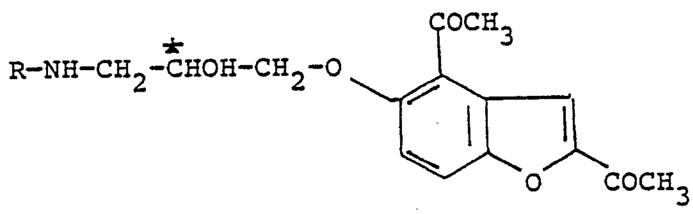

(I)

worin R eine Isopropyl- oder tert.Butylgruppe darstellt sowie von deren Säureadditionssalzen aus 2,4-Diacetyl-5-hydroxybenzofuran, dadurch gekennzeichnet, daß

a) 2,4-Diacetyl-5-hydroxybenzofuran der Formel

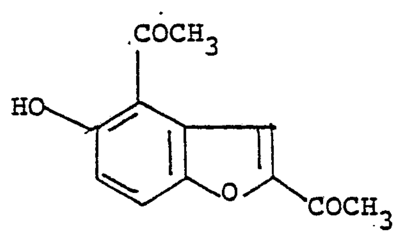

(II)

mit einem halogenierten Epoxid der Formel :

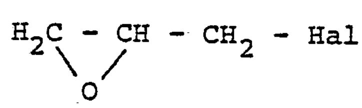

(III)

(worin Hal für Cl oder Br steht) zur Umsetzung gebracht wird, um das 2,4-Diacetyl-5-(2,3-epoxypropyloxy)-benzofuran der Formel

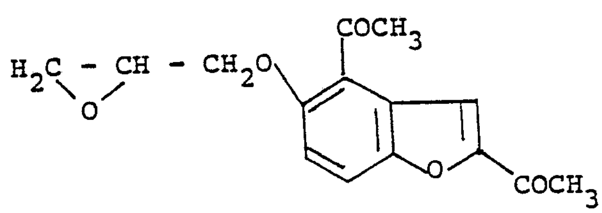

(IV)

zu erhalten, und danach

b) das so erhaltene Derivat IV mit einem Amin $NH_2R$ (worin R wie oben definiert ist) zur Umsetzung gebracht wird.

2. Verfahren nach Anspruch 1, dadurch gekennzeichnet, daß

a) 1,2 bis 1,8 Mol eines Epoxids der Formel III mit 1 Mol des substituierten Benzofurans der Formel II in einem $C_3$-$C_6$-Keton in Gegenwart eines Reagens, ausgewählt aus Li, Na, K, $Na_2CO_3$ und $K_2CO_3$, wenigstens 4 h lang unter Rückfluß reagieren gelassen wird ; und

b) 1,5 bis 2,2 Mol eines Amins der Formel $NH_2R$, worin R die in Anspruch 1 angegebene Bedeutung hat, mit 1 Mol der Verbindung der Formel IV in einem $C_1$-$C_3$-Alkohol wenigstens 2 h unter Rückfluß reagieren gelassen wird.

3. Verwendung der nach den Ansprüchen 1 und 2 erhältlichen neuen Verbindungen der allgemeinen Formel (I) zur Herstellung von therapeutischen, als cardioselektive β-Blocker wirkenden Zusammensetzungen, welch letztere zumindest eine neue Verbindung der Formel (I) gemeinsam mit einem physiologisch akzeptablen Exzipienten enthalten.